# EUROPEAN PATENT SPECIFICATION

(11) **EP 1 993 499 B1**
(45) Date of publication and mention of the grant of the patent: **16.01.2013**
(21) Application number: 07752670.5
(22) Date of filing: 08.03.2007
(51) Int. Cl.: A61F 13/44, A61F 13/36

(54) **X-RAY DETECTABLE ELEMENT FOR ASSOCIATION WITH SURGICAL ABSORBENT SUBSTRATES AND METHOD OF MAKING**
MITTELS RÖNTGENSTRAHLEN NACHWEISBARES ELEMENT ZUM EINSATZ MIT CHIRURGISCHEN ABSORPTIONSSUBSTRATEN UND HERSTELLUNGSVERFAHREN
ELEMENT DETECTABLE AUX RAYONS X POUR UNE ASSOCIATION AVEC DES SUBSTRATS ABSORBANTS CHIRURGICAUX ET PROCEDE DE FABRICATION

(30) Priority: 14.03.2006 US 782141 P
(43) Date of publication of application: 26.11.2008
(73) Proprietor: Covidien LP, Mansfield, MA 02048 (US)
(72) Inventor: PATEL, Harish A., Norfolk, MA 02056 (US); FINK, David, Franklin, MA 02038 (US)
(74) Representative: Gray, James
(86) International application number: PCT/US2007/005986
(87) International publication number: WO 2007/106373

(56) References cited:
- EP-A1- 0 201 348
- WO-A1-2004/004616
- US-A1- 2005 049 563
- US-A1- 2005 049 563
- US-A1- 2005 054 951
- US-A1- 2005 054 951
- US-A1- 2005 154 445
- US-A1- 2005 154 445

## Description

### CROSS-REFERENCE TO RELATED APPLICATIONS

This patent application claims priority to and the benefit of U.S. Provisional Patent Application No. 60/782,141, filed in the U.S. Patent and Trademark Office on March 14, 2006.

### TECHNICAL FIELD

The present disclosure relates to an x-ray detectable element, and more particularly, relates to an x-ray detectable element for use in association with surgical absorbent substrates.

### BACKGROUND

Radiopaque elements are associated with surgical absorbent substrates to assist in their detection, retrieval and accounting during surgical procedures. As appreciated, any foreign matter mistakenly left within a surgery site may cause serious complications during the patient's recovery. For example, a forgotten sponge left at the surgical site may lead to serious infection or death. Subsequent removal of a forgotten sponge requires the reopening of the surgery site. This reopening could lead to infection, further scarring, or other more serious complications. It is therefore necessary to provide an absorbent substrate that is capable of being detected prior to the closing of the surgery site.

### SUMMARY

Accordingly, the present disclosure relates to an x-ray detectable element for use in association with a surgical absorbent substrate. The detectable element has an extruded sheath at least partially encasing an elongated radiopaque core. The absorbent substrate may include, woven and non-woven, surgical sponges and gauze.

The radiopaque core of the x-ray detectable element is an elongated filament or thread containing anywhere from 30% to 90% opacifying agent or agents. The opacifying agent or agents may be combined, or blended, with one or more elastomeric materials, and/or polymers, to form the core. Such an elastomeric polymer is polyvinylchloride (PVC). A common opacifying agent is barium sulfate, BaSO₄. The cross-section of the radiopaque core is generally circular permitting for uniform visibility of the element under x-ray throughout the length of the element. Alternatively, the cross-section of the radiopaque core may be any shape, including square, rectangular and oval. The cross-section of the radiopaque core may also be lobed or multilobed. The radiopaque core may be of a continuous length within the sheath, or the radiopaque core may be intermittently spaced in the longitudinal direction for easier recognition over other structures in the surgical field, such a pacemaker wires.

The sheath of the x-ray detectable element is extruded about the radiopaque core, or both the radiopaque core and the sheath are extruded together, e.g., co-extruded. The sheath at least partial encases the radiopaque core. The sheath may be colored for improved visibility within a blood red environment. The sheath may also be composed of hydrophobic material so as to repel blood or other fluids encountered in the surgical field. The sheath may be anywhere from 80 % to 99 % elastomeric material.

The sheath may further be coated or impregnated with leachable microbial agents. Yet another variation of the element may have a sheath impregnated or coated with magnetic material for detection by an electro-magnetic device. Additional opacifying agent or agents may be added to the sheath for improved detectability.

The sheath may be radially partitioned into sheath regions in the longitudinal direction for improved visibility. One or more of the sheath regions may be of different colors and/or compositions. Sheath regions of different colors permit improved visibility of the element, while embedded opacifying agent or agents and magnetic material increase the detectability of the element. An additional layer at least partially encasing the sheath may also be added to the element for increased visibility. The additional layer may be composed of any combination of any or all of the above mentioned materials or colors.

### BRIEF DESCRIPTION OF THE DRAWINGS

Preferred embodiments of the present disclosure will be better appreciated by reference to the drawings wherein:
**FIG. 1A** is an axial cross-sectional view of an x-ray detectable element in accordance with the principles of the present disclosure;
**FIG. 1B** is a longitudinal cross-sectional view of the detectable element of **FIG. 1A****;**
**FIG. 2A** is an axial cross-sectional view of an alternate embodiment of the x-ray detectable element of **FIGS. 1A** and **1B****;**
**FIG. 2B** is a longitudinal cross-sectional view of the detectable element of **FIG. 2A****;**
**FIG. 3A** is an axial cross-sectional view of an alternate embodiment of the x-ray detectable element of **FIGS. 1A** and **1B****;**
**FIG. 3B** and **3C** are longitudinal cross-sectional views of the x-ray detectable element of **FIG. 3A** taken along the lines **b-b** and **c-c** of **FIG. 3A****,** respectively;
**FIG. 4A** is an axial cross-sectional view of an alternate embodiment of the x-ray detectable element of **FIGS. 1A** and **1B****;**
**FIG. 4B** is a side view of the x-ray detectable element of **FIG. 4A****;**
**FIG. 5** is an axial cross-sectional view of an alternate embodiment of the x-ray detectable element of **FIGS. 1A** and **1B****;**
**FIG. 6** is a longitudinal cross-sectional view of an alternate embodiment of the x-ray detectable element of **FIGS. 1A** and **1B****;**
**FIG. 7** is a longitudinal cross-sectional view of an alternate embodiment of the x-ray detectable element of **FIGS. 1A** and **1B****;**
**FIG. 8** is a longitudinal cross-sectional view of an alternate embodiment of the x-ray detectable element of **FIGS. 1A** and **1B****;**
**FIG. 9** is a fragmentary plan view of a surgical absorbent substrate in association with an x-ray detectable element constructed in accordance with the present disclosure;
**FIG. 10** is a fragmentary plan view of a surgical absorbent substrate in which an x-ray detectable element is formed as an integral part of the substrate; and
**FIGS. 11A** and **11B** are plan views of surgical absorbent substrates having differing patterns formed by x-ray detectable elements.

### DESCRIPTION OF THE PREFERRED EMBODIMENTS

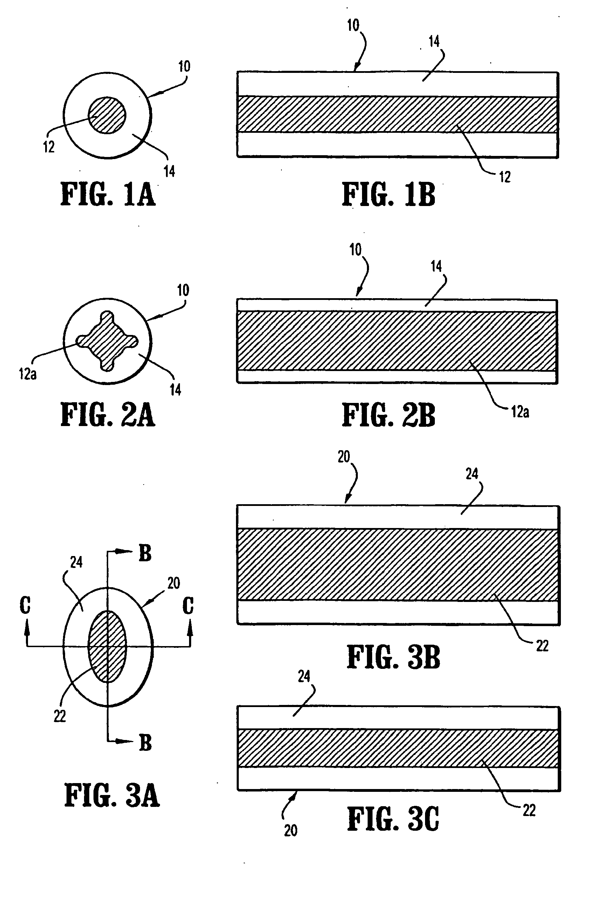

Referring now to the drawings wherein like reference numerals illustrate similar components throughout the several views, **FIGS. 1A** and **1B** illustrate one preferred embodiment of the present disclosure. X-ray detectable element **10** is contemplated for use in association with a surgical absorbent substrate, such as a sponge, gauze, or the like. X-ray detectable element **10** includes radiopaque core **12** partially encased within sheath **14.** Radiopaque core **12** is an elongated filament or thread containing, preferably, anywhere from 30% to 90% opacifying agent or agents. Other percentages for the opacifying agents are also contemplated. The opacifying agent or agents may be combined, or blended, with one or more other elastomeric materials or polymers to form radiopaque core **12.** Such an elastomeric polymer is polyvinylchloride (PVC). A common opacifying agent is barium sulfate, BaSO₄. In one preferred embodiment, radiopaque core **12** has a circular cross-section which provides uniform visibility of the element along its length when viewed under x-ray. Radiopaque core **12** may, however, take any form, including square, rectangular, oval, lobed and multi-lobed **12a (****FIGS. 2A** and **2B****).**

Sheath **14** of x-ray detectable element **10** at least partially encases radiopaque core **12** and, preferably, fully encloses radiopaque core **12.** Sheath **14** may be fabricated from an elastomeric material, polymeric material, or the like. Sheath **14** may be colored for improved visibility. For example, the colors of sheath **14** are chosen to contrast element **10** with linens and dressings found in the surgical field. Such colors are inclusive of blue or green, although other colors are contemplated. Sheath **14** may also be white to contrast element **10** with the blood present in the surgical site. Sheath **14** may also be composed of hydrophobic material so as to repel blood or other fluids encountered in the surgical field. Sheath **14** may comprise anywhere from 80% to 99% elastomeric material although other compositions are also envisioned. The percentage of elastomeric material used in sheath **14** alters the strength, elasticity, heat bondability to the absorbent substrate, and integrity of element **10.**

Sheath **14** may further be coated, or impregnated, with leachable microbial agents. Sheath **14** may also be impregnated, or coated, with magnetic material for detection by an electro-magnetic device. Additional opacifying agent or agents may be added to sheath **14** for improved x-ray detectability. It is further envisioned that x-ray detectable element 10 may be of a heat laminated design.

**FIGS. 3A-3C** show x-ray detectable element **20** with radiopaque core **22** having an oval cross-section within sheath **24.** **FIG. 3B** shows a longitudinal cross-sectional view of element **20** along plane **b-b** of **FIG. 3A. FIG. 3C** shows a longitudinal cross-sectional view of element **20** along plane **c-c** of **FIG. 3A****.** The larger dimension of radiopaque core **22** in plane **b-b** is more visible under x-ray than the smaller dimension of core **22** in plane **c-c.** X-ray detectable element **20** having oval shaped radiopaque core **22** may be associated with an absorbent substrate in such a manner so as to produce a uniquely visible profile under x-ray.

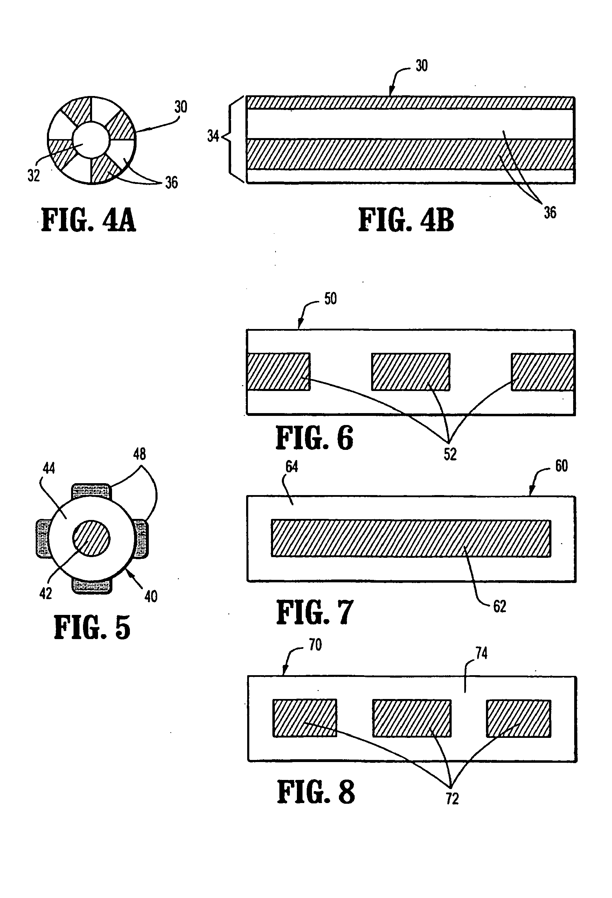

**FIGS. 4A** and **4B** illustrate an alternate embodiment of the present disclosure. X-ray detectable element **30** includes radiopaque core **32** and sheath **34** co-axially mounted about the radiopaque core **32.** Sheath **34** is preferably disposed about core **32** by extrusion, co-extrusion, or other methodologies familiar to those in the art. Elongated sheath **34** is portioned into radial segments or sheath regions **36** along the longitudinal axis. One or more sheath regions **36** may be of different colors and/or composition for increased visability and/or detectability. In one embodiment, elongated sheath **34** is radially partitioned into eight **(8)** sheath regions **36.** Sheath regions **36** may be alternating in colors, such as blue or green or white, for facilitating in enhancing visibility and/or detecting the presences of blood, as discussed herein above. Sheath regions **36** composed of additional opacifying agent or agents increase the detectability of element **30** under x-ray, while the addition of magnetic material to any of sheath regions **36** would make element **30** to be detected by an electro-magnetic device. Sheath **34** may have any number of sheath regions **36** possessing any or all of the aforementioned properties.

**FIG. 5** illustrates an alternate embodiment of the present disclosure. In accordance with this embodiment, x-ray detectable element **40** has radiopaque core **42,** sheath **44,** and an additional layer of material **48** partially encasing sheath **44.** Additional layer **48** may be composed of any or all of the above mentioned materials or colors. Additional layer **48** may provide for increased visibility and/or detectability of element **40.**

The radiopaque core may be of a continuous length within the sheath, as shown in **FIGS. 1-4****,** or the radiopaque core may be intermittently spaced in the longitudinal direction, as depicted in **FIG. 6****.** The intermittent radiopaque core **52** provides easier recognition of element **50** over other structures in the surgical field, such a pacemaker wires. In yet another embodiment, depicted in **FIG. 7****,** x-ray detectable element **60** may have radiopaque core **62** fully encased within sheath **64,** i.e., not extended to the ends of sheath **64.** In a further embodiment, depicted in **FIG. 8****,** x-ray detectable element **70** has intermittent radiopaque core **72** fully encased within sheath **74.**

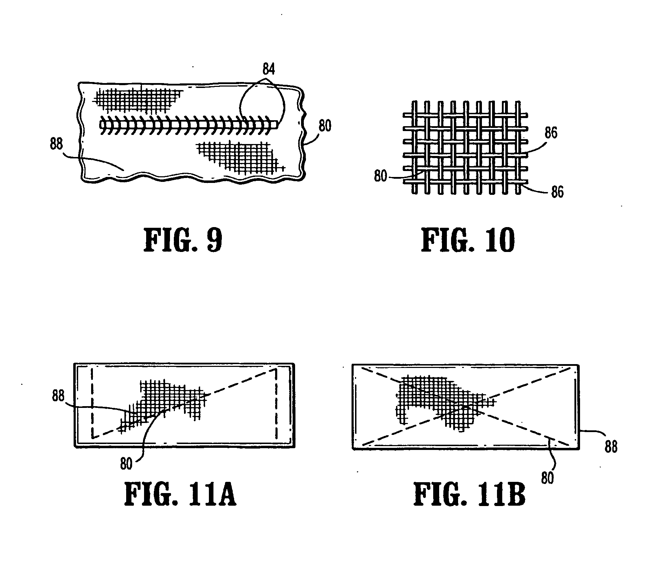

**FIGS. 9-11** illustrate x-ray detectable element **80** in association with absorbent substrate **88.** **FIG. 9** depicts element **80** secured on absorbent substrate **88** through the use of stitching **84.** **FIG. 10** shows x-ray detectable element **80** woven amongst fibers **86** of absorbent substrate **88.** Element **80** can be associated with absorbent substrate **88** by any of the aforementioned methods. Additionally, element **80** may be associated with substrate **88** through bonding and adhesion. FIGS. 11A and **B** show x-ray detectable element **80** woven into absorbent substrate **88** in various patterns for improved visibility and/or detectability. **FIG. 11A** depicts x-ray detectable element **80** woven into absorbent substrate **88** in the pattern of an extended "Z". **FIG. 11B** depicts element **80** woven into absorbent substrate **88** in an "X" pattern. Absorbent substrate **88** may be a sponge, gauze, or the like and may be fabricated from, woven and non-woven, cotton, synthetic fibers, or the like.

It will be understood that various modifications may be made to the embodiments disclosed herein. Therefore, the above description should not be construed as limiting, but merely as exemplifications of preferred embodiments.

In one embodiment the present apparatus for absorbing fluids in body tissue utilizes barium sulfate as the opacifying agent.

In another embodiment the present apparatus for absorbing fluids in body tissue includes a radiopaque core that defines a general oval cross-section.

In another embodiment the present apparatus for absorbing fluids in body tissue includes a radiopaque core that is fully encased within the sheath.

## Claims

1. An apparatus for absorbing fluids in body tissue, which comprises: an absorbent substrate (88); and
an elongated detectable element (50,60,70) associated with the substrate (88) and defining a longitudinal axis, the detectable element (50,60,70) having an elongated radiopaque core (52,62,72) and a sheath at least partially encasing the core (52,62,72), the core (52,62,72) defining a plurality of core sections arranged in spaced relation along the longitudinal axis.

2. An apparatus for absorbing fluids in body tissue, which comprises:
an absorbent substrate (88); and
an elongated detectable element (30) associated with the substrate (88) and defining a longitudinal axis, the detectable element (30) having an elongated radiopaque core (32) and a sheath (34) at least partially encasing the core (32), the sheath including a plurality of radial sheath regions (36), at least a first region of the sheath regions (36) comprising an opacifying agent.

3. An apparatus for absorbing fluids in body tissue, which comprises:
an absorbent substrate (88); and
a detectable element (10) associated with the substrate (88) and defining a longitudinal axis, the detectable element (10) having an elongated radiopaque core (12a) and a sheath (14) at least partially encasing the core (12), the elongated core (12a) defining a multi-lobal cross-sectional dimension, along the longitudinal axis.

4. The apparatus of claims 1 or 2 or 3 wherein the absorbent substrate (88) is a surgical sponge or gauze.

5. The apparatus of claims 1 or 2 or 3 wherein the radiopaque core (12a,32,52,62,72) comprises an opacifying agent.

6. The apparatus of claims 1 or 2 or 3 wherein the radiopaque core (12a,32,52,62,72) includes an elastomeric polymer.

7. The apparatus of claims 1 or 2 or 3 wherein the sheath (14,34) is colored.

8. The apparatus of claims 1 or 2 or 3 wherein the sheath (14,34) comprises an elastomeric material.

9. The apparatus of claims 1 or 2 or 3 wherein the sheath (14,34) comprises a hydrophobic material.

10. The apparatus of claims 1 or 2 or 3 wherein the sheath (14,34) comprises one or more leachable antimicrobial agents.

11. The apparatus of claims 1 or 2 or 3 wherein the sheath (14,34) comprises magnetic material.

12. The apparatus of claims 1 or 2 or 3 wherein the radiopaque core (12a,32,52,62,72) contains 30% to 90% opacifying agent.

13. The apparatus of claim 2 wherein the first and second regions of the radial sheath (36) regions are radially spaced with respect to the longitudinal axis.

14. The apparatus of claims 1 or 2 or 3 wherein the sheath (14,34) is an extruded sheath.

## Patentansprüche

1. Eine Vorrichtung zur Absorption von Flüssigkeiten im Körpergewebe, aufweisend:
ein absorbierendes Trägermaterial (88); und
ein längliches nachweisbares Element (50,60, 70), welches mit dem Trägermaterial (88) verbunden ist und eine Längsachse definiert, wobei das nachweisbare Element (50, 60, 70) einen länglichen radiopaken Kern (52, 62, 72) und einen Mantel aufweist, welche den Kern (52, 62, 72) zumindest teilweise umgibt, und der Kern (52, 62, 72) eine Vielzahl von Kernabschnitten umfasst, welche beabstandet entlang der Längsachse angeordnet sind.

2. Eine Vorrichtung zur Absorption von Flüssigkeiten im Körpergewebe, aufweisend:
ein absorbierendes Trägermaterial (88); und
ein längliches nachweisbare Element (30), welches mit dem Trägermaterial (88) verbunden ist und eine Längsachse definiert, wobei das nachweisbare Element (30) einen länglichen radiopaken Kern (32) und einen Mantel (34) aufweist, welche den Kern (32) zumindest teilweise umgibt, der Mantel eine Vielzahl von radialen Mantelbereichen (36) beinhaltet und zumindest ein erster Bereich der Mantelbereiche (36) ein Trübungsmittel aufweist.

3. Eine Vorrichtung zur Absorption von Flüssigkeiten im Körpergewebe, aufweisend:
ein absorbierendes Trägermaterial (88); und
ein nachweisbares Element (10), welches mit dem Trägermaterial (88) verbunden ist und eine Längsachse definiert, wobei das nachweisbare Element (10) einen länglichen radiopaken Kern (12a) und einen Mantel (14) aufweist, welche den Kern (12) zumindest teilweise umgibt, und der längliche Kern (12a) entlang der Längsachse eine Mehrflügel-Querschnittsfläche aufweist.

4. Vorrichtung nach Anspruch 1 oder 2 oder 3, wobei das absorbierende Trägermaterial (88) ein chirurgischer Schwamm oder Gewebe ist.

5. Vorrichtung nach Anspruch 1 oder 2 oder 3, wobei der radiopake Kern (12a, 32, 52, 62, 72) ein Trübungsmittel aufweist.

6. Vorrichtung nach Anspruch 1 oder 2 oder 3, wobei der radiopake Kern (12a, 32, 52, 62, 72) ein elastomeres Polymer beinhaltet.

7. Vorrichtung nach Anspruch 1 oder 2 oder 3, wobei der Mantel (14, 34) farbig ist.

8. Vorrichtung nach Anspruch 1 oder 2 oder 3, wobei der Mantel (14, 34) ein elastomeres Material aufweist.

9. Vorrichtung nach Anspruch 1 oder 2 oder 3, wobei der Mantel (14, 34) ein wasserabweisendes Material aufweist.

10. Vorrichtung nach Anspruch 1 oder 2 oder 3, wobei der Mantel (14, 34) ein oder mehrere auslaugbare antimikrobielle Substanzen aufweist.

11. Vorrichtung nach Anspruch 1 oder 2 oder 3, wobei der Mantel (14, 34) ein magnetisches Material aufweist.

12. Vorrichtung nach Anspruch 1 oder 2 oder 3, wobei der radiopake Kern (12a, 32, 52, 62, 72) 30% bis 90% Trübungsmittel beinhaltet.

13. Vorrichtung gemäß Anspruch 2, wobei die ersten und zweiten Bereiche der radialen Mantelbereiche (36) in Bezug auf die Längsachse radial beanstandet sind.

14. Vorrichtung nach Anspruch 1, 2 oder 3, wobei der Mantel (14, 34) ein extrudierter Mantel ist.

## Revendications

1. Appareil pour absorber des fluides dans un tissu corporel, qui comprend : un substrat absorbant (88) ; et
un élément détectable allongé (50, 60, 70) associé au substrat (88) et définissant un axe longitudinal, l'élément détectable (50, 60, 70) ayant une âme radio-opaque allongée (52, 62, 72) et une gaine englobant au moins partiellement l'âme (52, 62, 72), l'âme (52, 62, 72) définissant une pluralité de sections d'âme disposées de manière espacée le long de l'axe longitudinal.

2. Appareil pour absorber des fluides dans un tissu corporel, qui comprend : un substrat absorbant (88) ; et
un élément détectable allongé (30) associé au substrat (88) et définissant un axe longitudinal, l'élément détectable (30) ayant une âme radio-opaque allongée (32) et une gaine (34) englobant au moins partiellement l'âme (32), la gaine comprenant une pluralité de régions de gaine radiales (36), au moins une première région des régions de gaine (36) comprenant un agent opacifiant.

3. Appareil pour absorber des fluides dans un tissu corporel, qui comprend :
un substrat absorbant (88) ; et
un élément détectable (10) associé au substrat (88) et définissant un axe longitudinal, l'élément détectable (10) ayant une âme radio-opaque allongée (12a) et une gaine (14) englobant au moins partiellement l'âme (12), l'âme allongée (12a) définissant une dimension multilobée en section transversale, le long de l'axe longitudinal.

4. Appareil suivant la revendication 1, 2 ou 3, dans lequel le substrat absorbant (88) est une éponge chirurgicale ou de la gaze.

5. Appareil suivant la revendication 1, 2 ou 3, dans lequel l'âme radio-opaque (12a, 32, 52, 62, 72) comprend un agent opacifiant.

6. Appareil suivant la revendication 1, 2 ou 3, dans lequel l'âme radio-opaque (12a, 32, 52, 62, 72) comprend un polymère élastomère.

7. Appareil suivant la revendication 1, 2 ou 3, dans lequel la gaine (14, 34) est colorée.

8. Appareil suivant la revendication 1, 2 ou 3, dans lequel la gaine (14, 34) comprend une matière élastomère.

9. Appareil suivant la revendication 1, 2 ou 3, dans lequel la gaine (14, 34) comprend une matière hydrophobe.

10. Appareil suivant la revendication 1, 2 ou 3, dans lequel la gaine (14, 34) comprend un ou plusieurs agents antimicrobiens lessivables.

11. Appareil suivant la revendication 1, 2 ou 3, dans lequel la gaine (14, 34) comprend une matière magnétique.

12. Appareil suivant la revendication 1, 2 ou 3, dans lequel l'âme radio-opaque (12a, 32, 52, 62, 72) contient 30 % à 90 % d'agent opacifiant.

13. Appareil suivant la revendication 2, dans lequel les première et seconde régions des régions de gaine radiales (36) sont espacées radialement par rapport à l'axe longitudinal.

14. Appareil suivant la revendication 1, 2 ou 3, dans lequel la gaine (14, 34) est une gaine extrudée.
